# EUROPEAN PATENT SPECIFICATION

(11) **EP 1 549 340 B1**
(45) Date of publication and mention of the grant of the patent: **24.12.2008**
(21) Application number: 03730976.2
(22) Date of filing: 05.06.2003
(51) Int. Cl.: A61K 39/39

(54) **BIOFUNCTIONAL CPG OR OLIGO-/POLYNUCLEOTIDE AND TOXIN OR ENTEROTOXIN CONTAINING COMPOSITION**
BIOFUNKTIONALES CpG ODER OLIGO-/POLYNUCLEOTID UND TOXIN ODER ENTEROTOXIN ENTHALTENDE ZUSAMMENSETZUNG
COMPOSITION BIFONCTIONNELLE CONTENANT UN CPG OU UN OLIGO-/POLYNUCLEOTIDE ET UNE TOXINE OU UNE ENTEROTOXINE

(30) Priority: 05.06.2002 SE 0201701; 05.06.2002 US 385588 P
(43) Date of publication of application: 06.07.2005
(73) Proprietor: Duotol AB, 426 74 Västra Frölunda (SE)
(72) Inventor: HOLMGREN, Jan, S-426 74 Västra Frölunda (SE); HARANDI, Ali, M., S-431 69 Mölndal (SE)
(74) Representative: Nilsson, Brita Linnea
(86) International application number: PCT/SE2003/000935
(87) International publication number: WO 2003/103708

(56) References cited:
- WO-A2-01/83528
- WO-A2-02/11761
- WO-A2-99/61056
- MCCLUSKIE MICHAEL J. ET AL.: 'CpG DNA as mucosal adjuvant' VACCINE vol. 18, 2000, pages 231 - 237, XP000996190
- MCCLUSKIE MICHAEL J. ET AL.: 'Mucosal immunization of mice using CpG DNA and/or mutants of the heat-labile enterotoxin of escherichia coli as adjuvants' VACCINE vol. 19, 2001, pages 3759 - 3768, XP004241795
- MCCLUSKIE MICHAEL J. ET AL.: 'CpG DNA is a potent enhancer of systemic and mucosal immune responses against hepatitis B surface antigen with intranasal administration to mice' THE JOURNAL OF IMMUNOLOGY vol. 161, 1998, pages 4463 - 4466, XP002125530
- RANKIN R. ET AL.: 'CpG-containing oligodeoxynucleotides augment and switch the immune responses of cattle to bovine herpesvirus-1 glycoprotein D' VACCINE vol. 20, 2002, pages 3014 - 3022, XP004371831
- KLINMAN ET AL.: 'CpG motifs as immune adjuvants' VACCINE vol. 17, 1999, pages 19 - 25, XP000946061

### SEQUENCE LISTING

<110> Gotovax AB
<120> Bifunctional composition for modulating the immune system and its use
<130> 110082002
<150> US 60/385,588 and SE 0201701-0
   <151> 2002-06-05
<160> 4
<170> PatentIn version 3.1
<210> 1
   <211> 20
   <212> DNA
   <213> synthetic sequence
<400> 1
   tccatgacgt tcctgacgtt 20
<210> 2
   <211> 20
   <212> DNA
   <213> Murinae gen. sp.
<400> 2
   tccaggactt ctatcaggtt 20
<210> 3
   <211> 24
   <212> DNA
   <213> synthetic sequence
<400> 3
   tcgtcgtttt gtcgttttgt cgtt 24
<210> 4
   <211> 24
   <212> DNA
   <213> Homo sapiens
<400> 4
   tgctgctttt gtgcttttgt gctt 24

## Description

The present invention relates to a bifunctional composition for modulating the immune system and its use. More precisely, the invention relates to a bifunctional composition comprising an immunomodulating nucleic acid component, optionally in combination with a specific antigen, conjugated with a specific cell-surface binding protein component. The composition is useful for treatment of tumors, infections, graft rejections, immunosuppressive states, autoimmune diseases and allergies, as well as immunoprophylaxis, immunotherapy or induction of tolerance, and for treatment *ex vivo* of an antigen-presenting cell for subsequent infusion into a mammal for vaccination or immunotherapy purposes.

### Background

Unmethylated CpG in bacterial DNA or synthetic oligodeoxynucleotides (ODNs) are known as potent activators of the immune system and inducers of a variety of Th1-associated immunomodulatory cytokines suggesting a possible utility for enhancing innate immunity against infectious pathogens (Krieg, 2001).

More recently, other nucleic acid sequences with immunoinhibitory rather than stimulating properties have been described that could potentially be used for suppressing harmful immune reactions such as in different immunopathological conditions including autoimmune diseases, allergies and graft rejections.

It has been well documented for the immunopotentiating CpG motif-containing nucleic acid sequences, and it is now believed this will also be the case for immunoinhibitory sequences, that these sequences will modulate both the magnitude and characteristics of innate immune responses as well as of adaptive immune responses to specific antigens. Such adaptive immune responses that can be enhanced, suppressed or otherwise modulated by specific nucleic acid sequences may comprise many different groups of antigens. Examples of such antigens include, but are not limited to pathogen-derived antigens, mammalian tissue-derived antigens, allergens and host-derived antigens, administered either together with the immunomodulating nucleic acid sequence or being already present in the host at the time for the administration of the latter sequence such as is the case, for instance, for pathogen-derived antigens in an ongoing infection and mammalian tissue-derived antigens in an ongoing autoimmune disorder or after transplantation of non-syngenic organs, tissues or cells. In some instances, it has been described that coupling of an immunopotentiating nucleic acid sequence to a specific antigen is a particularly efficient way of stimulating a specific adaptive immune response to the antigen in question, supposedly by increasing the chance of uptake of both the specific antigen and the immunostimulatory nucleic acid sequence by the same (antigen-presenting) cells (Shirota *et al.*, 2001; Shirota *et al.*, 2000).

One particularly important aspect of immune modulation relates to specific antigens and immunomodulating agents administered at a mucosal surface rather than by parenteral injection; examples of such mucosal routes of administration include oral or gastrointestinal administration, intranasal administration, intrarectal administration and administration at a genital mucosa. It is known that administration of antigens with or without additional specific immunomodulating agents can either induce an immune response at the mucosal surface (and occasionally also systemically) or specifically suppress systemic immune responses to selected antigens by inducing peripheral tolerance (often referred to as "oral tolerance" when induced by the oral route of mucosal administration). There have been many recent efforts to exploit this type of mucosally induced immunosuppression/immunomodulation for immunotherapeutic purposes in various autoimmune, allergic or immunopathologic conditions. A particularly efficient means of administering such peripheral tolerance described is to administer a specific tolerogenic antigen in conjunction with the non-toxic binding subunit of cholera toxin (CTB) (Holmgren *et al.*, 2003). Another relevant application of immunological agents for either stimulating, suppressing or otherwise modulating innate immunity and adaptive immune responses is through application onto or into the skin. Subcutaneous and intracutaneous administration of vaccines and other antigens are long-established procedures, and more recently it has also been described that topical application directly onto the skin (so called transdermal immunization) can also be used to either stimulate immune responses or suppress or immunodeviate specific responses.

To our knowledge there is no prior art disclosing either products based on or the use of bifunctional immunomodulating compositions comprising an intracellularly effective immunomodulating nucleic acid component, optionally in combination with a specific antigen, associated with a specific cell-surface binding protein component, wherein the latter component serves to specifically increase the uptake and/or reactivity of the other, intracellularly effective immunomodulating nucleic acid component and optionally the specific antigen.

Thus, to our knowledge, there is also no prior art disclosing a method of prophylactic or therapeutic treatment of epithelial tumors or non-respiratory epithelial infections in a mammal by local administration of an antigen-free pharmaceutical preparation comprising at least one unmethylated 5'-cytosine, guanine-3' dinudeotide-containing immunostimulatory oligo- or polynucleotide (ISS) selected from single stranded and double stranded DNA, single and double stranded RNA and modified polynucleotides, in association with a specific cell-surface binding protein component for local treatment of epithelial tumors or non-respiratory epithelial infections in a mammal.

### Description of the invention

The present invention provides in one aspect a preferably synergistic bifunctional immunomodulating composition (BIC) of an intracellularly acting immunostimulatory, immunoinhibitory or otherwise immunomodulating nucleic acid sequence (in the future referred to as immunomodulating sequences, IS), optionally in combination with a specific antigen, conjugated with a specific cell surface binding protein component serving to increase the uptake and/or the reactivity of the IS component and optionally said specific antigen.

Thus, the present invention is directed to a bifunctional composition comprising an intracellularly effective immunomodulating nucleic acid component containing at least one unmethylated 5'-cytosine, guanine-3' dinucleotide (CpG) selected from an oligonucleotide or a polynucleotide with either a natural phosphodiester backbone a phosphothioate backbone or a chimera of phosphodiester and phosphothioate backbone optionally in combination with a specific antigen, conjugated with a protein binding to specific receptors on mammalian cell surfaces selected from the group consisting of cholera toxin B subunit(CTB), *Escherichia coli* heat labile enterotoxin B subunit (LTB), and proteins or protein derivatives that react with antiserum to CTB or LTB, bind to GM1 ganglioside.

In an embodiment of the bifunctional composition the immunomodulating nucleic acid component is selected from the group consisting of an unmethylated 5'-cytosine, guanine-3' dinucleotide-containing immunostimulatory oligo- or polynucleotide (ISS) selected from single stranded and double stranded DNA, single and double stranded RNA and modified polynucleotides.

In another embodiment the ISS is selected from nucleotide sequences having at least 6 bases or base pairs and optionally comprising phosphorothioate backbones.

In a presently preferred embodiment the ISS is derived from a microbial genome, such as *Herpes simplex* virus genome.

In a further presently preferred embodiment the ISS is 5'-purine purine(pyrimidine) CG pyrimidine pyrimidine -3' (5'-Pu Pu(Py)CGPy Py-3'), e.g. 5'-GACGTT-3' or 5'-GTCGTT-3'.

In yet another embodiment the optionally present specific antigen is in fact present and is selected from the group consisting of synthetic or natural pathogen-derived antigens, mammalian tissue-derived antigens, allergens and host-derived antigens.

Another aspect of the invention is directed to the bifunctional composition of the invention for use as a medicament.

In a further aspect of the invention the bifunctional composition of the invention is used for the manufacture of a pharmaceutical preparation for the prophylactic or therapeutic treatment of tumors, infections, graft rejections, immunosuppressive states, autoimmune diseases and allergies.

In an embodiment of this aspect of the invention the pharmaceutical preparation is antigen-free and is for local treatment of epithelial tumors or non-respiratory epithelial infections in a mammal.

In another embodiment of this aspect of the invention the composition of the invention comprising a specific antigen is used for the manufacture of a pharmaceutical preparation for immunoprophylaxis, immunotherapy or induction of tolerance. Such a composition is also used for treatment *ex vivo* of an antigen-presenting cell for subsequent manufacture of a pharmaceutical preparation for infusion into a mammal for vaccination or immunotherapy purposes.

The invention may be used in a method of prophylactic or therapeutic treatment of infections, tumors, autoimmune disorders, allergies, graft rejections and other immunopathological or immunosuppressed conditions, comprising administering to a mammal an effective amount of the bifunctional immunomodulating composition described above (in the future referred to as "bifunctional immunomodulating complex", BIC) through either a mucosal, a skin-based, a deeper parenteral, or an intraamniotic route of administration, whether given alone or in combination with a specific antigen, or through administration of cells, which have been treated with BIC ex vivo before infusion into the mammal.

Thus, the invention may be useful in a method of prophylactic or therapeutic treatment of tumors, infections, graft rejections, immunosuppressive states, autoimmune diseases or allergies in a mammal comprising the step of administering a prophylactically or therapeutically effective dose of a pharmaceutical preparation according to the invention to said mammal.

In an embodiment of the invention the pharmaceutical preparation is antigen-free and the treatment is for epithelial tumors or non-respiratory epithelial infections in said mammal, and the administration is local administration thereof to a site of epithelial tumor or non-respiratory epithelial infection in said mammal.

The invention is also useful in a method of immunoprophylaxis, immunotherapy or induction of tolerance in a mammal comprising the step of administering a prophylactically or therapeutically effective dose of a composition according to the invention comprising a specific antigen to said mammal.

The invention is further useful in a method of treatment *ex vivo* of an antigen-presenting cell with a composition according to the invention comprising a specific antigen for subsequent infusion into a mammal for vaccination or immunotherapy purposes.

It should be understood that the pharmaceutical preparations, and the bifunctional compositions as such, may comprise inert diluents, excipients, and other components necessary agents for systemic or mucosal administration, e.g. as described in the US or European Pharmacopoeia.

The term "intracellularly effective immunomodulating nucleic acid component" is used to describe that the immunomodulating nucleic acid component alters or modulates the immune response induction through effector signals intracellularly.

A prophylactically or therapeutically effective dose of a pharmaceutical preparation used in the present invention is to be determined by an attending physician or veterinary with the guidance of instructions from the manufacturer, the mammal in question, the size of the mammal and other criteria commonly known by a man skilled in the art.

Even though the present invention is predominantly illustrated by description of embodiments concerning investigations of immunostimulatory IS containing at least one methylated 5'-cytosine, guanine-3' dinucleotide (CpG)-containing immunostimulatory IS chemically linked to CTB, the claimed scope of protection is supported by the following reasoning.

The concept of immunostimulatory DNA was born almost two decades ago when it was described that DNA purified from bacteria could inhibit the growth of various animal tumors, augment natural killer (NK) cell activity and induce IFN-α/β and -γ from mouse spleen cells and human peripheral blood lymphocytes. Since then, many investigations have revealed that unmethylated CpG with appropriate flanking regions (CpG motifs) are responsible for the stimulatory effects of bacterial DNA on vertebrate immune systems. Interactions between unmethylated CpG motifs in bacterial DNA or in synthetic oligonucleotide (ODN) and toll-like receptor-9 (TLR-9) receptors in antigen-presenting cells (APCs), such as dendritic cells and macrophages, rapidly, through the toll/IL-1-receptor signaling pathway, stimulate the cells to produce proinflammatory Th1 polarizing cytokines, such as IFN-γ, IL-1-β and IL-12, to upregulate costimulatory molecules on the APCs and to activate B-cells for proliferation, antibody production and IL-6 secretion (Holmgren *et al.*, 2003; Krieg, 2001; Krieg, 2002).

Parenteral delivery of immunostimulatory CpG DNA, in the absence of antigen, has been demonstrated to induce non-specific Th1-like innate immune responses of a protective nature against infections caused by several different pathogens. This conceptual frame at the systemic level was recently extended to mucosal innate immunity by findings that a single mucosal-vaginal administration of immunostimulatory CpG ODN, in the absence of any antigen, elicits rapid production of Th1-associated cytokines IFN-γ, IL-12 and IL-18 and of CC chemokines RANTES, MiP-1α and MiP-1β in the mouse female genital tract and/or the genital lymph nodes (Harandi *et al.*, 2003). In addition to the impact of CpG DNA on the induction of innate immune responses, a number of studies have also shown that CpG DNA is also working as a vaccine adjuvant. The majority of studies using CpG DNA as an adjuvant have been carried out with a systemic route of administration; however mucosal immunization with CpG DNA as adjuvant has recently also been shown to elicit both mucosal and systemic antigen-specific immune responses (Holmgren *et al.*, 2003).

Several variations of the original CpG DNA composition have also proved to provide useful IS. For instance, several variations from the natural phosphodiester backbone have indeed not only functioned but have provided additional potency to the IS. The latter effect is probably mainly by stabilizing the IS from degradation, but in some instances, such as shown for unmethylated DNA with a phosphothioate backbone the ODN has been shown to have inherent immunostimulatory activity even in the absence of any associated CpG motif by acting as a chemoattractant on APCs (Baek *et al*., 2001). Examples of useful backbones described include, but are not limited to different phosphodiester or phosphothioate backbones and to chimeras of these two types.

Also variations in the immunostimulatory sequences of IS have been described allowing for either optimization of their efficacy in different animal species, provision of immunoinhibitory rather than immunostimulatory activity, and/or having differential immunomodulating activity resulting in a skewing of the immune response towards either a Th1 type or a Th2 type of response. With regard to different sequences being optimal for different species, it is well documented that the optimal CpG motif for stimulating immune responses in mice differs from that in humans; in humans a few different active sequences have been identified and also that the flanking regions around the critical CpG motifs further determine the optimal activity on different types of APC. When it comes to immunoinhibitory instead of immunostimulatory IS, is well documented that methylated DNA and ODNs can have immunoinhibitory rather than stimulatory activity. Along the same line it has been shown that ODNs containing either of the four possible single-nucleotide bases can inhibit cytokine production from and activation and maturation of APCs provided, however, that the backbone is phosphothioate (Zhu *et al.*, 2002). It has been also demonstrated that placement of two Guanosines immediately after CG dinucleotides resulting in (three consecutive Guanosines) would convert a stimulatory ODNs to immunoinhibitory ODNs (Stunz *et al*., 2002). Thus, depending on both sequence and backbone structure, DNA can inhibit as well as stimulate immune responses. Further, randomly synthesized ODNs without CpG motifs have also been described as adjuvants for the induction of Th2 differentiation, whereas ODNs containing CpG motifs induce strong Th1-skewed responses (Sano *et al.*, 2003).

Similar to what has been described for the IS component of the BIC, also the specific cell-binding component can be modified or varied. In the best studied example, CTB, any structural modification that would not significantly affect the GM1 ganglioside receptor binding activity of the molecule or its stability should be able to replace the parent CTB molecule, as would also be the case for the analogous, closely related binding subunit protein of the heat-labile enterotoxin of *E. coli* (LTB).

In the examples given, the two components of BIC, as exemplified by CpG ODN and CTB, were linked to each other by coupling technology based on chemical modifications of both of the components, for the IS using thiolation and for CTB maleimide activation. A number of alternative chemistries for linking different types of molecules together exist and are well known in the art and are therefore not further expanded upon here. Likewise, methods are well known in the art of linking two molecules together through a spacer molecule to which latter category can also be included an immunologically active protein or other molecule comprising or including a specific antigen.

The present invention is also useful in methods for stimulating, suppressing or otherwise modulating innate immunity and/or antigen-specific acquired (adaptive) immune responses. For these purposes, the BIC can be administered to a mammal in an effective amount by either of a number of different routes. It can be given by any of many possible mucosal routes, including but not being limited to the oral route, the gastrointestinal route, the rectal route, the genital-mucosal route, an intransasal route, a respiratory-inhalation route, a conjunctival mucosal route or a middle-ear mucosal route. The BIC can also be given by a skin-related route, such as epicutaneously on the skin surface, intradermally or subcutaneously. The BIC can further be given by a deeper parenteral administration, including but not being limited to an intravenous injection, an intramuscular injection, an intraperitoneal injection, an intraaminotic injection, an intrathecal injection, an intraarticular injection, and a targeted lymph node injection. Yet another administration of BIC could be in the form of cells, such as APC, which have been treated with the BIC ex vivo before being administered to a mammal by either of the systemic or mucosal routes mentioned above.

The invention also relates to use of BIC for prophylactic or therapeutic treatment of infections or other diseases. In the examples provided, BIC is used for the treatment prophylactically or therapeutically of a viral infection caused by herpes simplex type 2 virus (HSV-2), but depending on the type of BIC used and its route of administration, the potential for prophylactic or therapeutic treatment extend to a very large number of infections caused by viruses, bacteria, fungi or protozoans and other parasites; different types of tumors; different types of autoimmune diseases and allergies; graft reactions including transplanted organs, tissues or cells; and different types of other immunological conditions including also different types of immunosuppressive states (e.g. immunodepression caused by HIV infection).

The invention will now be exemplified by the following description of the drawings and embodiments, but it should be understood that the scope of protection is not intended to be limited to the details described. Further, the teachings of the citations referred to are incorporated herein by reference.

### Description of the drawings

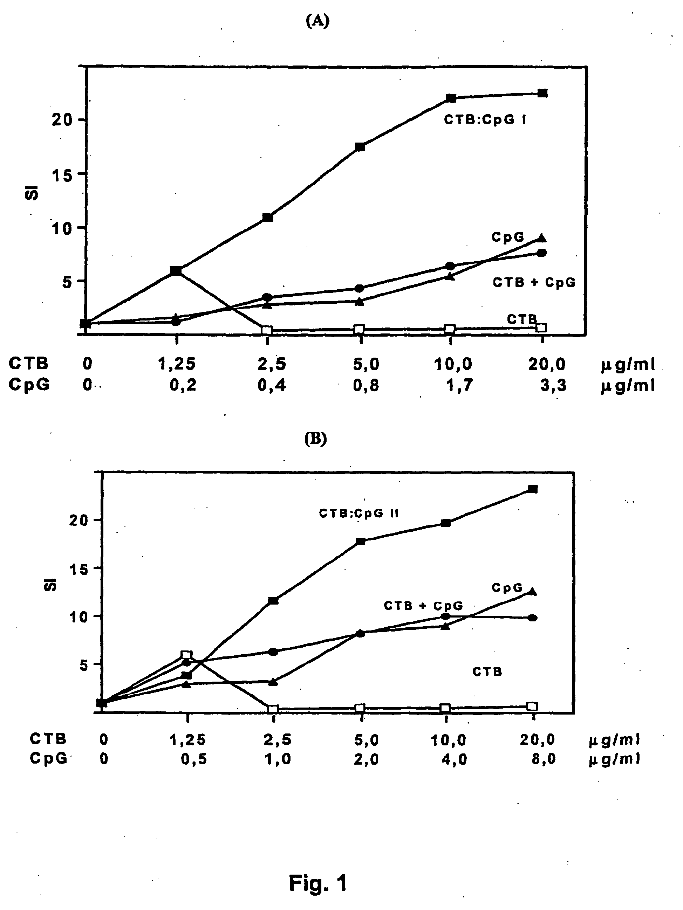

**Figure 1** shows two diagrams (A) and (B) of proliferative responses of murine spleen cells after in vitro stimulation with increasing concentrations of either CTB, CpG ODN, admixture of CTB and CpG or CTB:CpG conjugates.

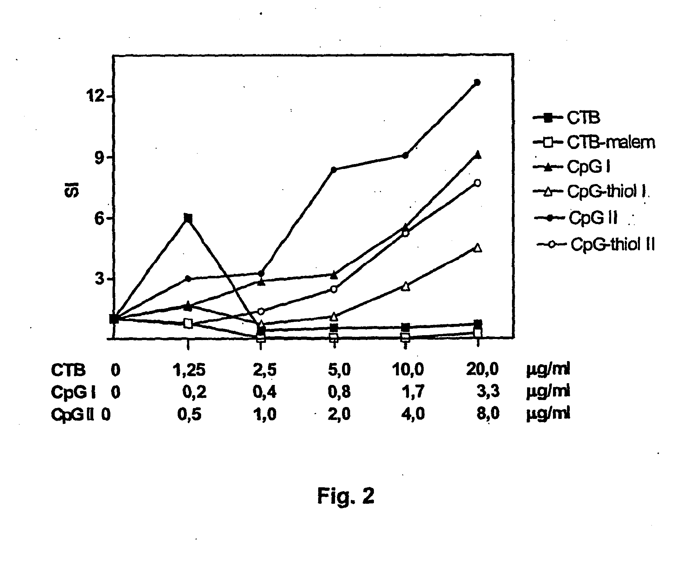

**Figure 2** shows a diagram of maleimide-activation of CTB or thiolation of CpG ODN does not affect the proliferative response. Murine spleen cells were cultivated in the presence of increasing concentrations of either CTB, maleimide-activated CTB, CpG ODN or thiol-modified CpG ODN for 72h and then the cells were harvested and proliferative responses were examined as described in Materials and Methods The data are expressed as Stimulation Index (Sl).

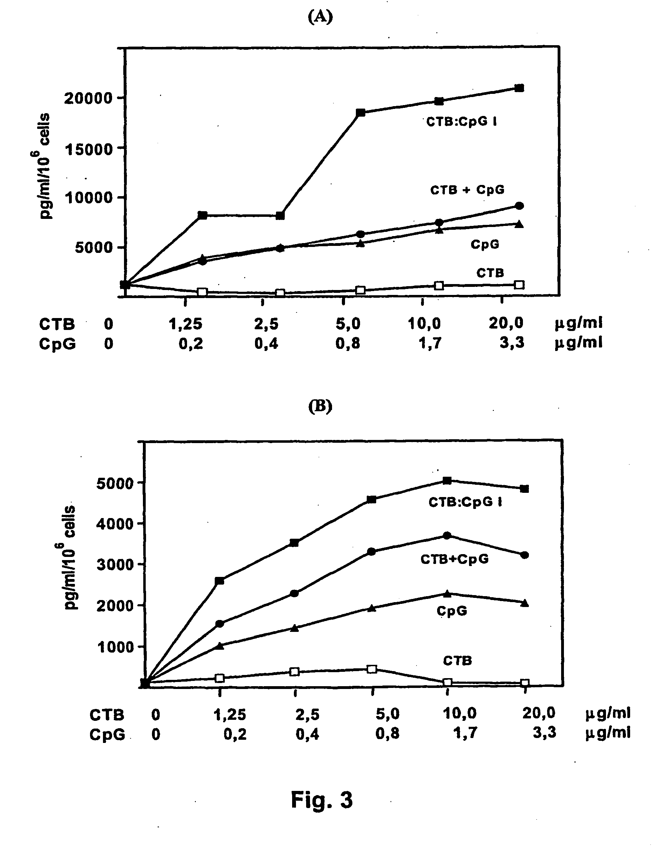

**Figure 3** shows two diagrams (A) and (B) of production of IP-10 following stimulation of murine spleen cells with CTB:CpG I, recCTB, CpG ODN or admixture of recCTB and CpG ODN. Spleen cells from naive C57bI/6 mice were cultivated in the presence of different concentrations of either CTB:CpG I, recCTB, CpG ODN or admixture of recCTB and CpG ODN and the supernatants were subjected to IP-10 ELISA.(a) 24h. (b) 48h. (The data is representative of two different experiments).

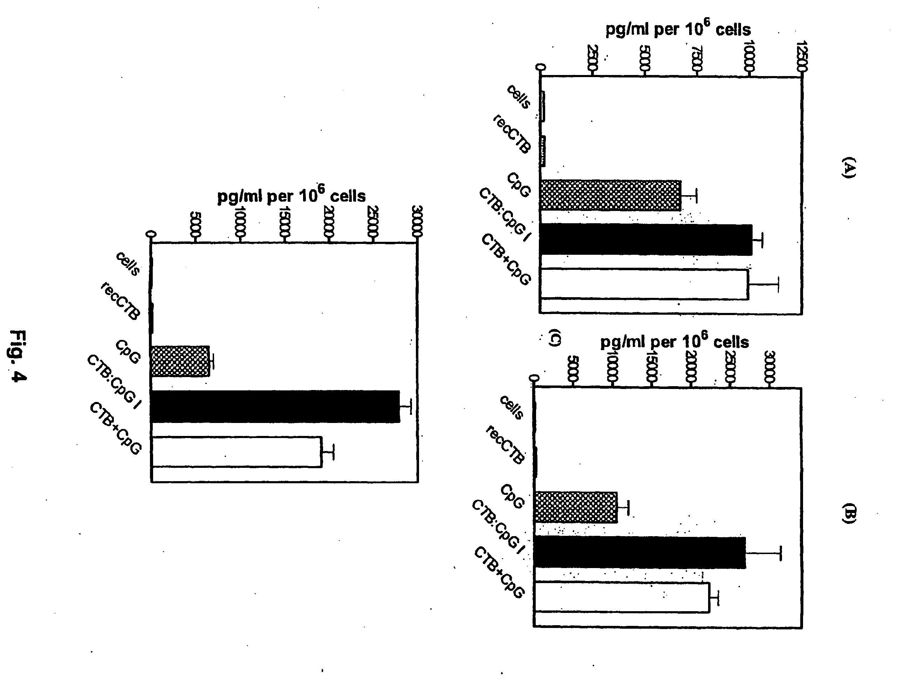

**Figure 4** shows three diagrams (A), (B) and (C) of production of MIP-1α by murine spleen cells cultivated in the presence of either CTB:CpG I [20µg/ml], recCTB [20µg/ml], CpG ODN [3,3µg/ml] or admixture of recCTB and CpG ODN [20µg/m+3,3µg/ml]. Data are expressed as the mean and standard error of the mean. (a) 24h. (b) 48h. (c) 72h. Data are pooled from two different experiments.

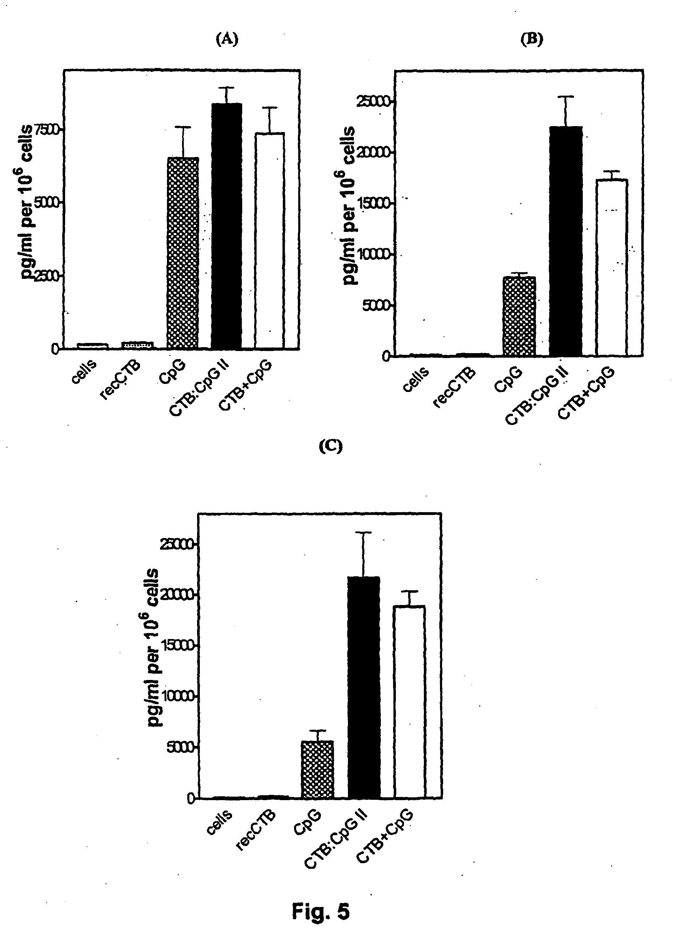

**Figure 5** shows three diagrams (A), (B) and (C) of production of MIP-1α in supernatants from proliferation of pooled spleen cells, cultivated in the presence of either CTB:CpG II [20µg/ml], recCTB [20µg/ml], CpG ODN [8,0µg/ml] or admixture of recCTB and CpG ODN[20µg/ml+8,0µg/ml].Data are expressed as the mean and standard error of the mean. (a) 24h. (b) 48h. (c) 72h. Data are pooled from two different experiments.

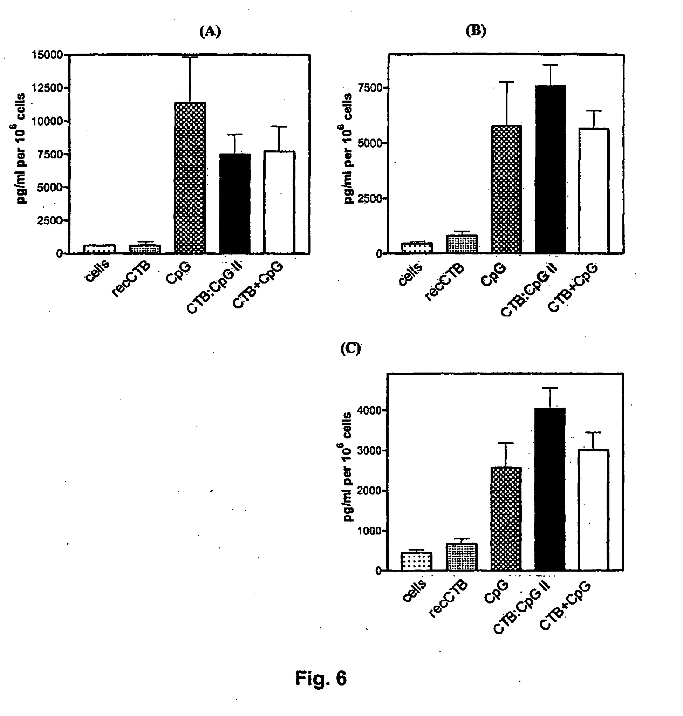

**Figure 6** shows three diagrams (A), (B) and (C) of RANTES production by murine spleen cells cultivated in the presence of either CTB:CpG I [20µg/ml], recCTB [20µg/ml], CpG ODN [3,3µg/ml] or admixture of recCTB and CpG ODN [20µg/m+3,3µg/ml]. Data are expressed as the mean and standard error of the mean. (a) 24h. (b) 48h. (c) 72h. Data pooled from two different experiments.

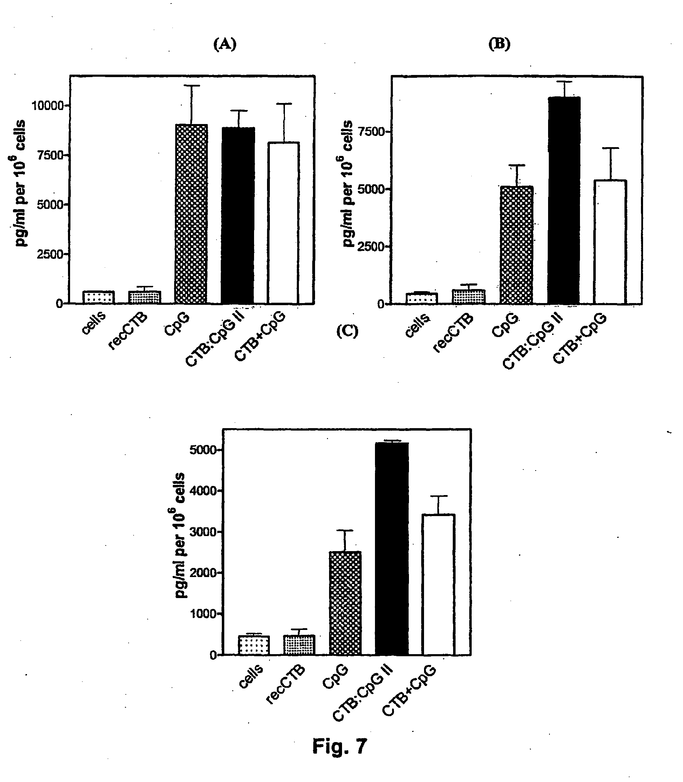

**Figure 7** shows three diagrams (A), (B) and (C) of RANTES production by murine spleen cells cultivated in the presence of either CTB:CpG II [20µg/ml], recCTB [20µg/ml], CpG ODN [8,0µg/ml] or admixture of recCTB and CpG ODN[20µg/ml+8,0µg/ml]. Data are pooled from from two different experiments and expressed as the mean and standard error of the mean. (a) 24h. (b) 48h. (c) 72h

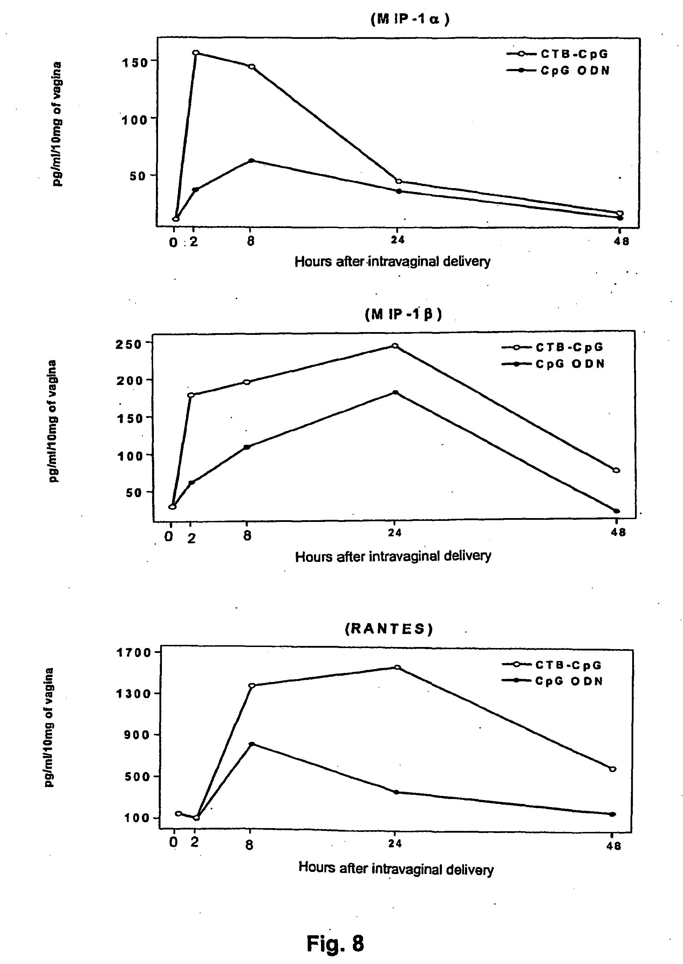

**Figure 8** shows three diagrams of the time course of CC chemokine production in the mouse vagina after intravaginal administration of CpG ODN or CTB:CpG. Groups of naive mice were pretreated with DP. Six days later, they were delivered intravaginally with CpG ODN or CTB:CpG and sacrificed at different time points. The vaginas were excised and saponin extracted and the chemokine contents examined using ELISA. The data are shown as µg/ml per 10 mg of the vagina.

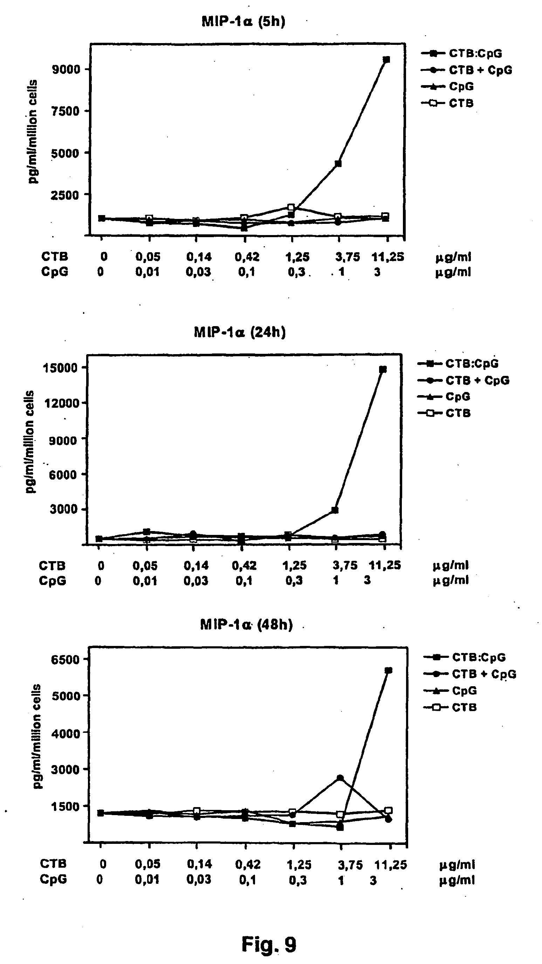

**Figure 9** shows three diagrams of the time course of MIP-1α production in the supernatants of human PBMCs cultured in the presence of different concentrations of CpG ODN, CTB, admixture of CpG ODN or CTB:CpG. Concentrations of CpG ODN and CTB correspond to the concentrations used in the conjugate.

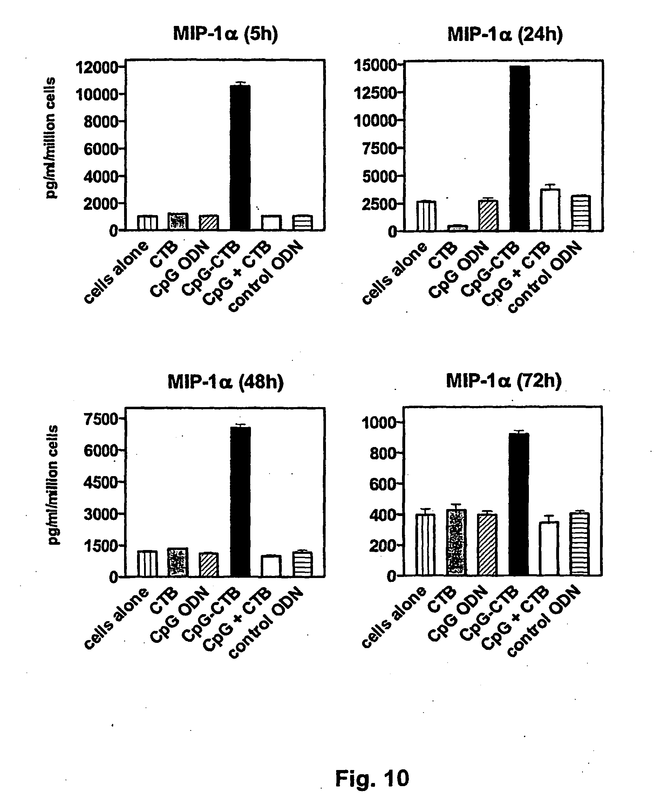

**Figure 10** shows four diagrams of MIP-1α production in the supernatants of human PBMCs cultured in the presence of CTB (11,25 µg/ml), CpG ODN (3 µg/ml), admixture of CpG ODN and CTB, CTB:CpG or non-CpG control ODN (3 µg/ml). Data are expressed as the mean and the standard error of the mean.

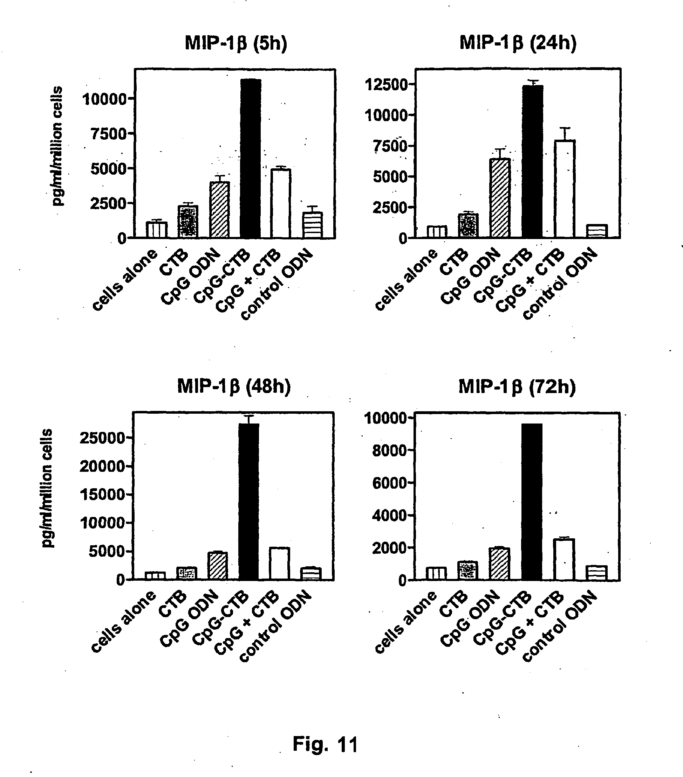

**Figure 11** shows four diagrams of MIP-1β production of supernatants from human PBMCs cultured with CTB (11,25 µg/ml), CpG ODN (3 µg/ml), admixture of CpG ODN (3 µg/ml) and CTB (11,25 µg/ml) CTB:CpG (11,25 µg/ml) or non-CpG control ODN (3 µg/ml). Data are expressed as the mean and standard error of the mean.

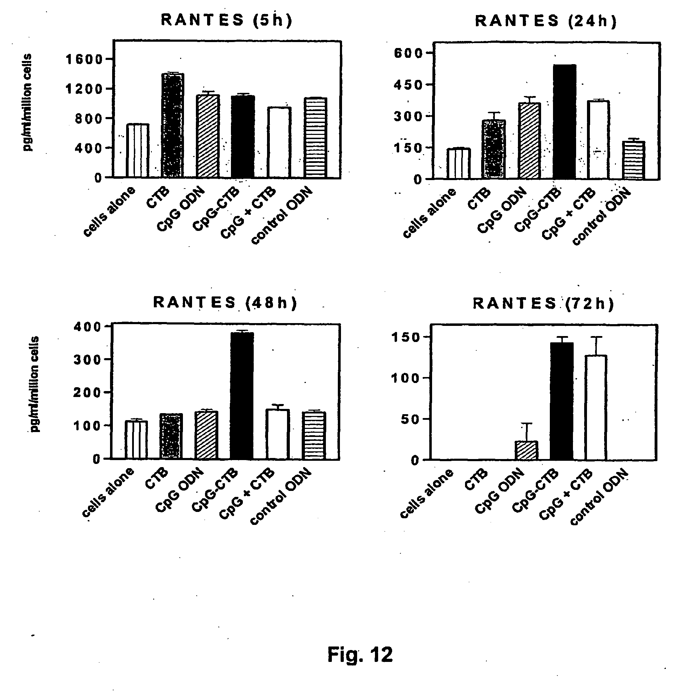

**Figure 12** shows four diagrams of RANTES production by human PBMCs cultured in the presence of CTB (11,25 µg/ml), CpG ODN (3 µg/ml), admixture of CpG ODN (3µg/ml) and CTB (11,25 µg/ml) on non-CpG control ODN (3µg/ml). Data are expressed as man and standard error of the mean.

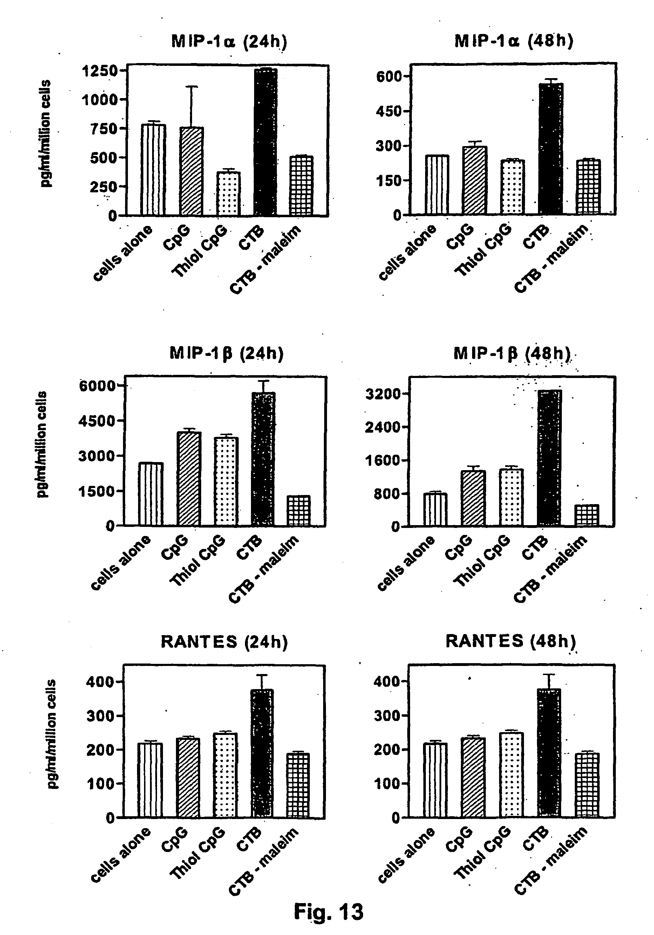

**Figure 13****.** shows six diagrams of production of CC chemokines (MIP-1α, MIP-1β and RANTES) in the supernatants of human PBMCs cultured in the presence of CTB (11,25µg/ml), CTB-maleimide (11,25 µg/ml), CpG ODN (3 µg/ml) or Thiol CpG ODN (3 µg/ml). Data are expressed as the mean and the standard error of the mean.

### Description of embodiments

As a background to the disclosed embodiments involving CpG and female genital tract mucosal immunity against herpes simplex virus (HSV) infection, the following brief descriptions of these entities is presented.

Bacterial DNA that has a relative abundance of CpG motifs and CpG oligonucleotides can activate immune responses. *In vitro,* CpG ODN directly stimulate splenocytes, monocytes, dendritic cells, and macrophages to secrete a variety of cytokines such as IFN-γ [Yamamoto, 1992], IL-1β [Lipford, 1997], tumor necrosis factor-α (TNF-α) [Sparwasser, 1997], IL-12 [Lipford, 1997] and IL-18 [Roman, 1997], and activate B cells for IL-6 secretion and proliferation [Yi, 1996]. Further, CpG activates NK cells, macrophages [Sparwasser, 1997], B cells [Krieg, 1995] and dendritic cells [Sparwasser, 1998] to up-regulate major histocompatibility complex (MHC) class I, MHC class II, as well as co-stimulatory molecules such as CD80 and CD86. *In vivo*, systemic administration of CpG ODN was demonstrated to promote NK activity [Yamamoto, 1992], to increase the B cell percentage in the spleen [Krieg, 1995], and to increase IFN-γ [Yamamoto, 1992], IL-6 [Yi, 1996], TNF-α [Sparwasser, 1997] and IL-12 [Zimmermann, 1998] plasma levels or mRNA expression in tissues. The ability of CpG ODN to prevent malaria and *Listeria Monocytogenes* infection in animal model of the disease have recently been documented [Germazinski, 2001; Elkins, 1999; Krieg, 1998].

In addition to their effect on innate immune response, CpG ODN were shown to potentiate the specific immune responses to co-administered antigens [Ban, 2000]. Several recent reports have established the potent adjuvant activity of CpG ODN in vaccination with various antigens and with DNA vaccines in different animal model of the diseases [for review see [Krieg, 2001]. Of note, reports of the first human clinical trials demonstrated that CpG ODN co-administration with antigen is immunostimulatory and can be well tolerated. Despite the documented impact of CpG ODN on systemic immune response, little is known about the effect of CpG DNA on induction of immunity in the mucosal surfaces. It has been recently shown that a single vaginal administration of CpG ODN elicits strong Th1-assocaied cytokines and chemokine responses in the murine female genital tract mucosa (Harandi, et al 2003).

HSV-2 is a sexually transmitted pathogen that infects the human genital tract mucosa and is the most common case of genital ulcer disease in humans. The prevalence of HSV-2 infection is high in many countries, with 10-50% of the adult female population being infected [Kinghom, 1994], and the incidence is increasing worldwide [Nahmias, 1990]. Acquisition of HSV-2 infection is usually the consequence of transmission by genital contact. Acute genital herpes infection is characterized by brief period of intensive virus shedding into vaginal secretions, which is important for the transmission of the virus. Genital ulcers are common but disseminating disease and life-threatening complications such as meningitis may also occur in immunocompromized individuals. In early pregnancy, HSV-2 can cross the placental barrier and affect the fetus which may lead to a spontaneous abortion or serious damage to the fetus, including mental retardation [Whitley, 1991]. Thus, there remains a serious need to develop preventive strategies for genital herpes infection particularly since genital ulcers facilitate the transmission of the human immunodeficiency virus.

We investigated the impact of CpG ODN - CTB BIC without antigen co-administration on induction of local genital immunity and on prevention of a sexually transmitted pathogen, HSV-2. The results illustrate the potent preventive potential of CpG ODN-CTB BIC against genital herpes infection as a model system for sexually transmitted viral diseases.

### Materials and Methods

### Mice

Female 6 to 8 weeks old C57BI/6 mice were used for all experiments. The mice were purchased from M&B and kept in ventilated cages under specific-pathogen-free conditions at the EBM Animal Facility, Sahlgrenska Academy, Göteborg University. All experiments were performed with the approval from the Ethical Committee for Animal Experimentation in Göteborg, Sweden.

### ODNs

The ODNs were purchased from Cybergene AB (Novum Research Park, Sweden) or Qiagen. The CpG ODN used for murine studies was TCC ATG ACG TTC CTG ACG TT (SEQ ID NO: 1), a 20-mer with a nuclease-resistant phosphorothioate (PS) backbone that contains two copies of GACGTT motif. The murine control ODN was TCC AGG ACT TCT CTC AGG TT (SEQ ID NO: 2), a 20-mer with a nuclease-resistant phosphorothioate backbone that contains no CpG motif. For human in vitro tests, CpG ODN used was TCGTCGTTTTGTCGTTTTGTCGTT (SEQ ID NO: 3) a 24-mer with a nuclease-resistant phosphorothioate (PS) backbone that contains three copies of GTCGTT motif. The human control ODN used was TGCTGCTTTTGTGCTTTTGTGCTT (SEQ ID NO: 4), a 24-mer with a nuclease-resistant phosphorothioate backbone that contains no CpG motif.

### Chemical linking of the optimal murine and human CpG ODNs to CTB

Conjugation of CTB to either murine or human CpG ODN was done in three steps:

### a) Deprotection of disulphide linkage of thiol CpG ODN:

To cleave linkage of thiol-modified CpG ODNs (Qiagen), the ODNs were mixed with dithiotheritol (DTT) (Sigma) and incubated for either 90 min at room temperature (mouse construct I and human conjugate) or 16 h at 37°C (mouse construct II). After this, deprotected thiol-modified CpG ODNs were subsequently purified by gel filtration through Sephandex G 25 (DNA grade column 0.9 x 21.6 cm Pharmacia Amersham Biosciences) and subsequently eluted with 0,1M phosphate buffer, 0.15M NaCl and 0.01 M EDTA at pH 7.2. The absorbance of purified thiol-modified CpG ODN was read at 260n.m by a spectrophotometer.

### b) Maleimide activation of CTB:

Introduction of maleimide groups to CTB molecules was achieved by incubation with excess of sulfosuccinimidyl-4-(*N*-maleimidomethyl)cyclohexane-1-carboxylate (SMCC) (Pierce Chemicals) for 90 min at room temperature followed by purification by filtration through G 25 (PD 10 Pharmacia) and elution with 2ml 0,1M phosphate buffer, 0.15M NaCl and 0.01 M EDTA at pH 7.2.

### c) Conjugation of CTB to CpG ODN:

Maleimide-activated CTB and deprotected thiol-modified CpG ODNs were incubated together overnight at room temperature and then concentrated through Viva spin 5000 MWCO. The concentrated material was separated on Superdex 200 10/30 and the high molecular weight fraction was collected and further concentrated through 5000 MWCO. The conjugates were subjected to SDS-PAGE. CTB and CpG ODN were visualised with Coomassie Blue and SYBR GREEN, respectively. The GM1-binding capacity of conjugates was determined by GM1-ELISA.

### Murine proliferation assay

Spleen cells from naive C57BI/6 mice were isolated by passing the organs through nylon net. Erythrocytes were lysed by incubation of the cell with ammonium chloride (NH₄Cl) in 37°C for 10 min, after this cells were washed and stained with trypan blue and viable cells were counted in a büchner chamber. The cells were seeded in 96-wells flat bottom plate (Nunc) in triplicates (2 x 10⁵ cells/well) in lscove's medium supplemented with L-glutamine, 50 µM 2-mercaptoethanol, gentamicin, and 10% fetal calf serum. The cells were incubated at 37°C alone or in the presence of different concentrations of either recCTB, CTB-maleimide, CpG ODN, CpG-thiol, control ODN, admixture of recCTB and CpG ODN, admixture of CTB-maleimide and CpG-thiol, or CTB:CpG conjugates. The concentrations of rec CTB and CpG ODN used were corresponding to their concentrations in the conjugates. After 24, 48 and 72 h of incubation, culture supernatants were collected and assayed for chemokines contents. On day 3, cells were pulsed with 1 µCi of [³H]thymidine (Amersham Pharmacia) for 6-8 h and then the cellular DNA was harvested on glass fibre filters and then incorporation of the radioactive thymidine was assayed in a beta-counter in counts per minute (cpm). Data are expressed as stimulation index (SI), corresponding to the mean cpm for cells cultured with the substances divided by the mean cpm for cells alone.

### Intravaginal treatment

Mice were pretreated by subcutaneous (s.c.) injection with 3.0 mg of Depo-Provera (Upjohn s.a., Puurs, Belgium) in 150 µl of phosphate-buffered saline (PBS). Six days later, the mice were anaesthetised with isofluran (Baxter Medical AB) and administered ivag. with either rec CTB or 1µg of CpG ODN either in free form or linked to CTB (CTB:CpG conjugate) or left untreated.

### Extraction of chemokines from the vaginas

Extraction of chemokines from the vagina was performed by using a modified version of a PERFEXT method (Johansson, 1998). Briefly, mice were sacrificed at different time points after intravaginal delivery and the vaginas were excised and weighed before storage at -20°C in a PBS solution containing 2 mM phenylmethylsulfonyl fluoride, 0.1 mg of soybean trypsin inhibitor (Sigma) per ml, and 0.05 M EDTA. For PGE₂ assay, indomethacin (Sigma) was added to the tissue samples. The tissue samples were thawed and then permeabilized with saponin (Sigma) at a final concentration of 2% (wt/vol) in PBS at 4°C overnight. The tissue samples were then centrifuged at 13 000 rpm for 5 min and the supernatants and the sera were analysed for chemokines contents by using ELISA.

### Virus and virus challenge

HSV-2 strain 333 [Seth, 1974] were grown and titrated in cell monolayers of African green monkey kidney cells (GMK-AH1) and prepared by one cycle of freeze/thaw and subsequent removal of cellular debris by centrifugation. For HSV-2 challenge, each mouse was injected subcutaneously with 3.0 mg of DP in PBS. Six days later, the mice were anaesthetized using isofluran (Baxter Medical AB, Sweden) and challenged by intravaginal inoculation of 9 × 10⁴ PFU of a virulent HSV-2 strain in 10µl Hanks balanced salt solution (HBSS).

### Monitoring of infection

a. Viral replication. Following intravaginal HSV-2 infection, vaginal fluids were collected by pipetting 40µl of sterile HBSS in and out of the vagina until a discrete clump of mucus was retrieved and then a second wash was performed. The two washes were pooled and stored at -70°C. HSV-2 titers were determined by plaque assay on GMK-AH1 cell monolayers using standard method.

b. Inflammation and disease. Mice were examined daily for vaginal inflammation, neurological illness and death after HSV-2 infection. The severity of disease was graded as 0: healthy, 1: genital erythema, 2: moderate genital inflammation, 3: severe and purulent genital lesion, 4: hind-limb paralysis and 5: death or sacrifice due to paralysis

### Human PBMC proliferation assay

Human PBMCs were separated from healthy adults' blood samples by using Ficoll-Hypaque gradient centrifugation. The cells were seeded into flat bottom 96-well plates in Iscove's medium, supplemented with L-glutamin, 50 µM 2-mercaptoethanol, gentamicin and fetal calf serum in the presence of increasing concentrations of CTB, CpG ODN, admixture of CTB and CpG ODN or human CTB-CpG conjugate. The concentrations of CTB and CpG ODN correspond to their concentrations In the conjugate. Supernatants were collected at 5, 24, 48 and 72 hours and examined for their contents of CC or CXC chemokines. After 72 hours the cells were pulsed with 1 uCi of [³H]thymidine (Amersham Pharmacia) and incubated for 6-8 hours. The cellular DNA was harvested on glass fibre and the incorporation of the radioactive thymidine was assayed in a beta-counter in counts per minute (cpm). Data are expressed as stimulation index (SI), corresponding to the mean cpm for cells cultured with the substances divided by the mean cpm for cells alone.

### Chemokines quantification

Concentrations of RANTES, MIP-α, MIP-1β, MIP-2 and IP-10 in the tissue extracts and the culture supernatants were determined by using ELISA kits from R&D Systems (Abingdon, United Kingdom) or PeproTech EC Ltd (for IP-10) according to the manufacturer's recommendations.

### EXAMPLES

### EX.1 Construction of CTB:CpG conjugates

To link CpG ODN to CTB, to potentiate the immunostimulatory effect of CpG ODN, thiolated CpG ODNs were chemically conjugated to maleimide activated CTB. For deprotection of thiol-modified CpG, two approaches were used, which resulted in generation of two constructs of murine CTB:CpG conjugates, namely CTB:CpG I and CTB:CpG II as described in Materials and Methods. For human, only one CTB:CpG construct was made (see Materials & Methods). The conjugates were run on SDS-PAGE and stained with Coomassie Blue (protein staining) and SYBR GREEN II (DNA staining) which confirmed successful conjugation (Data not shown).

The two murine conjugates differ in CTB and CpG contents. Thus, the ratios of CTB to CpG were 6 and 2.5 for CTB:CpG I and II, respectively. The conjugates were examined for their GM1 binding property in GM1 ELISA.

### EX.2 Conjugation of CpG ODN to CTB potentiates the CpG ODN-induced proliferative response of murine splenocytes

Spleen cells from naive C57BI/6 mice were cultured with different concentrations of either rec CTB, CpG ODN, control ODN, CTB:CpG I or CTB:CpG II.

### CTB:CpG I

As shown in Figure 1a, spleen cells cultivated with the highest concentration of the conjugate (20 µg/ml) resulted in a SI of 22,6 while CpG ODN at a corresponding concentration (3,3µg/ml) resulted in only a SI of 9,1. The lowest concentration of the conjugate (1,25µg/ml) stimulated the proliferation to the same extent as the highest concentration of either CpG ODN alone or admixture of CTB and CpG. RecCTB had no stimulating effect on proliferation when used at any concentrations (Fig 1a). Control ODN at the same concentrations as CpG ODN had no effect on the proliferation (data not shown).

### CTB:CpG II

As for conjugate I, conjugate II at the highest concentration had a more stimulating effect on the proliferation (SI = 23,3) than CpG ODN alone (SI =12,6) or admixture of CTB and CpG (SI = 9,9). When the concentration of the conjugate was lowered to 2,5 µg/ml the same or even stronger proliferative response (SI = 12) was detected as compared with highest concentration of either CpG ODN or admixture of CTB and CpG ODN. CTB alone had no stimulating effect on proliferation when used at any concentrations (Fig 1b).

Proliferation shown as SI after 72h proliferation of pooled spleen cells from two naive C57bI/6 mice, cultivated with either conjugated CTB:CpG I, CTB:CpG II, CpG ODN, recCTB or admixture of recCTB and CpG ODN at different concentrations. In both fig (a) and (b) the concentration of recCTB and CpG ODN are corresponded to the concentrations of them in the conjugates.

### (a) CTB:CpG I. (b) CTB:CpG II.

To test if the activation of the CTB with maleimide or the coupling of a thiol to CpG ODN may have any effect on the spleen cell proliferation, spleen cells were cultivated in the presence of different concentrations of the maleimide-activated CTB or the thiolated CpG ODN.

Not at any of the different concentrations the coupling of a thiol group to CpG ODN or maleimide-activation of CTB resulted in higher level of proliferation than CpG ODN or CTB alone (Fig 2). Cultivation of spleen cells with admixture of CTB-malemid and CpG-thiol had no additional effect on the proliferation as compared with CpG ODN alone (data not shown).

### EX. 3 Conjugation of CpG ODN to CTB potentiates the CpG ODN-induced CXC and CC chemokine responses of murine splenocytes

To evaluate the effect of conjugation of recCTB to CpG ODN on induction of chemokine production, spleen cells were cultured with either CTB:CpG I, CTB:CpG II, recCTB, CpG ODN or admixture of recCTB and CpG ODN. At different time points supernatants were examined for the contents of CXC and CC chemokines.

### CXC Chemokines (exemplified by IP-10)

**CTB:CpG I.** In supernatant collected after 24 h culture of the spleen cells in complete medium 1250 pg/ml/10⁶ cells of IP-10 was detected that dramatically declined after 48 h (122,5 pg/ml/10⁶ cells). Cultivating of the spleen cells with CTB:CpG I resulted in an induction of IP-10 production. The production increased with increasing concentration of the conjugate. The highest concentration of conjugate used (20 µg/ml) resulted in a 20-fold and a 40-fold increasion in production of IP-10 at 24h and 48h as compared with those of untreated cells, respectively.

Much lower level of IP-10 was detected when spleen cells were cultivated with either CpG ODN alone or admixture of CTB and CpG ODN.
At 24h, lowest concentration of CTB:CpG I (1,25 µg/ml) induced more production of IP-10 than the highest concentrations of either CpG ODN alone or an admixture of recCTB and CpG ODN.

Not any of the tested concentrations of recCTB did induce IP-10 production at any time points examined (Fig 3a &3b).

**CTB:CpG II.** The concentration of IP-10 in cells cultivated alone was 1000 pg/ml /10⁶ cells at both 24 and 48 h. At 24 h, CpG ODN alone or admixture of CTB and CpG ODN induced the same level of IP-10 production (7-8-fold increase). Importantly, production of IP-10 was increased by 16-fold within 24 h after CTB:CpG II treatment. The IP-10 production was further increased at 48 h (18-fold increase). After 48 h, both CpG ODN alone and admixture of CTB and CpG ODN produced much lower levels of IP-10 as compared with the conjugates. RecCTB alone did not induce the IP-10 production at any time points examined. **MIP-2**

No MIP-2 production was detected in the supernatants of CpG ODN treated spleen cells.

### EX. 4 CC Chemokines (exemplified by MIP-1α and RANTES) MIP-1α

Low levels of MIP-1α (122,5-150 pg/ml/10⁶ cells) were found in the supernatants taken from cells alone at 24h, 48h and 72h. RecCTB did not induce any production of MIP-1α at any time points (Fig 4 and 5).

**CTB:CpG I.** As shown in figure 5, cultivation of spleen cells with CTB:CpG I increased the production of MIP-1α by 70-fold at 24 h and 180-fold at 48 h and further to a 186-fold at 72 h. At 24 h , however admixture of recCTB and CpG ODN induce production of MIP-1α to the same extent as the conjugate. The production of MIP-1α was decreased with time, yet showing 130-fold increase at 72h. Cells treated with CpG ODN alone did not produce comparable levels. At 48h CpG ODN induced a 73-fold increase in MIP-1α production that decreased to 50-fold within 72 h. RecCTB did not induce any appreciable levels of MIP-1α production at any time points tested (Fig 4).

**CTB:CpG II.** Cultivation of spleen cells with CTB:CpG II induced a 55-fold increase in MIP-1α at 24 h and 150-fold increase at 48 h that stayed at this level until 72 h. Admixture of recCTB and CpG ODN induced the production of MIP-1α (50-fold), this induction increased to 120-fold at 48 h and stayed up until 72h. At 48h, CpG ODN induced increase in MIP-1α production by 50-fold, that decreased to 40-fold at 72 h (Fig 5).

### RANTES

As for MIP-1α, low levels of RANTES (450- 600 pg/ml/10⁶ cells) were found in supernatant taken from cells alone at 24 h, 48 h and 72h. RecCTB did not induce production of MIP-1α at any time points (Fig 6 and 7).

**CTB:CpG I.** In the supernatants taken at 24 h, CpG ODN induced high level of RANTES (11000 pg/ml/10⁶ cells), the amount of RANTES produced by cells cultivated with either CTB:CpG I or admixture of recCTB and CpG ODN were lower. At 48 and 72 h, the levels of RANTES produced by CpG ODN treated cells decreased, whereas the levels of RANTES produced by cells treated with conjugate remained unchanged for at least 72 h (Fig 6).

**CTB:CpG II.** Comparable levels of RANTES was detected at 24 h in supernatants of CTB:CpG II, CpG ODN alone or admixture of recCTB and CpG ODN pulsed spleen cells. After 48 h, CTB:CpG II still produced the same level of RANTES whereas the production of RANTES in the others groups decreased. After 72 h, the production of RANTES was highest in the supernatants of cells cultivated with the conjugate (fig 7).

### EX. 4CTB:CpG conjugate induces strong CC chemokine responses in the murine female genital tract mucosa

To examine the effect of the conjugation of CTB to CpG ODN on chemokine responses in the female genital tract, groups of mice were treated with progesterone followed by a single intravaginal administration of either CTB, CpG ODN, or CTB:CpG conjugate. At different time points following the adminstration, the vaginas were taken and after saponin extraction, the levels of CC chemokines MIP-1α, MIP-1β and RANTES were determined.

### MIP-1α

MIP-1α was detected in low level in the vagina of naïve mice (12 pg/ml/10 mg of the vagina). Intravaginal administration of CTB did not induce MIP-1α production at any time point examined. Intravaginal CpG ODN administration increased the level of MIP-1α by 5-fold within 8 h, which then decreased and got back to the basal level within 48 h. Within 2 h a rapid 13-fold increase of MIP-1α was observed in mice treated with CTB:CpG conjugate, which stayed up for 8 h (12-fold increase) and declined within 24 h (4-fold) and reached the basal level by 48 h (figure 8).

### MIP-1β

MIP-β was detected in the vagina of naïve mice in low levels (30 pg per ml/10 mg of the vagina). Intravaginal administration of CTB did not induce MIP-1β production at any time point examined. Intravaginal CpG ODN treatment led to a 4-fold increase of MIP-1β within 8 h, which went up within 24 h (6-fold increase). The production of MIP-1β decreased and got back to basal level by 48 h. Intravaginal CTB:CpG treatment caused a rapid 6-fold increase in MIP-1β production within 2 h followed by a peak at 24 h (8-fold increase), which went down within 48 h (3-fold increase) (figure 2).

### RANTES

In the vagina of naïve mice low level of RANTES was detected (140 pg/ml/10 mg of the vagina). Intravaginal administration of CTB did not induce RANTES production at any time point examined. Intravaginal administration of CpG ODN increased the level of RANTES by 6-fold within 8 h, which decreased by 24 h (3-fold increase) and returned to the basal level within 48 h. Within 8 h after CTB:CpG administration, the level of RANTES was increased by 10-fold which stayed up for 24 h (11-fold increase) and then declined by 48 h (4-fold increase) (figure 8).

### EX. 5 Conjugation of CpG ODN to CTB dramatically increases the protective immunity against genital herpes infection

We have previously shown that a single vaginal administration of CpG ODN (60µg/mouse) without antigen codelivery elicits strong mucosal protective immunity in the murine female genital tract mucosa against genital herpes infection. Having shown that conjugation of CTB to CpG ODN elicits stronger proliferative and chemokine responses in vitro, we sought to examine the impact of the conjugation of CTB to CpG ODN on induction of protective immunity against genital HSV-2 infection. To this end, groups of DP-treated female C57BI/6 mice were administered intravaginally with 1µg of CpG ODN either singly or in the conjugate form with CTB, 24h prior to a vaginal challenge with a normally lethal dose of HSV-2. A group of mice was treated with rec CTB. Control as well as CTB-treated mice started to show macroscopic signs of the disease as early as 3 days following the HSV-2 challenge and had died as a result of neurological illness within 8 days of infection. Similarly, all mice administered intravaginally with 1mg of CpG ODN showed macroscopic signs of the disease and died within 12 days. Intravaginal administration of CTB:CpG conjugate, on the other hand, conferred 80% protection against HSV-2-induced death. This data indicates that vaginal-mucosal administration of minute amount of CTB:CpG conjugate confers strong protective immunity against genital herpes infection.

### EX. 6 Effect of the conjugation of CTB to CpG ODN on proliferative and chemokine responses by human peripheral blood mononuclear cells (PBMC)

To evaluate the effect of conjugation of CTB to CpG ODN on proliferation and chemokine productions by human PBMCs. PBMCs were cultured with either CpG ODN, CTB, CTB:CpG or admixture of CpG ODN and CTB. Supernatants were collected at different time points and examined by ELISA for their contents of chemokines. The cells were harvested after 72 h of culture for proliferation. The data shown are representative of the 3 experiments.

### a) conjugation of CpG ODN to CTB has no additional effect on the CpG ODN-induced proliferative response of human PBMCs

PBMCs were cultivated with different concentrations of either CTB, CpG ODN, admixture of CpG ODN and CTB, control ODN or CTB:CpG conjugate. After 3 days of incubation, the proliferative response was examined by using standard method (see material and methods). The PBMCs cultivated in the presence of different concentrations of CTB did not give rise to any detectable proliferative response. The treatment of the PBMCs with CpG ODN gave rise to a strong proliferative response. Proliferative response was observed when the PBMCs were cultivated in the presence of the three highest concentrations of CpG ODN (3 µl/ml, 1 µg/ml or 0,3 µg/ml). Cultivation of the PBMCs with CTB:CpG did not have any additional effect on CpG ODN-induced proliferative response. As expected, non-CpG control ODN induced a weak proliferative response.

### b) CC chemokine responses

### MIP-1α

As shown in figure 10, PBMCs cultivated with either CpG ODN, CTB, admixture of CpG ODN and CTB or control ODN at different concentrations did not induce MIP-1α production at any time point examined. In contrast, CTB:CpG showed increased level of MIP-1α within 5 h when a concentration of 3,75 µg/ml of CTB:CpG used (4-fold increase), that stayed up for 24 h (6-fold increase) and then went back to basal level at 48 h (figure 9). When a concentration of 11,25 µg/ml of CTB:CpG were used the level of MIP-1α was increased by 10-fold at 5 h, which further increased by 24 h (29-fold increase) and then declined by 48 h (6-fold increase) (figure 9-10). At 72 h, the levels of MIP-1α in CTB:CpG treated cells was low, yet showing higher levels compared to the other groups (data not shown).

Having shown that the highest concentration of CTB:CpG is optimal for induction of chemokine production, we then used the highest concentrations of the conjugate or the corresponding reagents for the following experiments.

### MIP-1β

In the supernatants of the PBMCs cultivated in the complete medium, a basal level of MIP-1β was detected. Treatment of the PBMCs with CTB or non CpG control ODN did not induce MIP-1β production at any time point examined (figure 11). The PBMCs cultivated with CpG ODN or mixture of CTB and CpG produced similar levels of MIP-1β within 5 h (4-fold), which increased within 24 h (7- and 9 fold increase) and declinecd by 72 h (3-fold increase). In supematans of cells incubated with CTB:CpG a 10-fold increase in MIP-1β production was observed at 5 h, that peaked at 48 h (23-fold increase) and stayed relatively high for at least 72 h (13-fold increase) (figure 11).

### RANTES

A basal level of RANTES was detected in supernatants of the PBMCs cultivated alone. Cultivation of the PBMCs in the presence of different reagents did not induce any increased level of RANTES at 5 h. Within 24 h, the level of RANTES in the supernatants of the cells incubated with CpG ODN or mixture of CpG and CTB increased by 3-fold, which returned to the basal level within 48 h. Incubation of the cells with CTB:CpG led to a 4-fold increase in RANTES production, which stayed up for 48 h (3-fold) and returned to the basal level by 72 h (figure 12).

### c) CXC chemokine responses

### IP-10

No detectable level of IP-10 was observed in the supernatants of the PBMCs incubated with either CpG ODN, CTB, mixture of CTB and CpG ODN, or CTB:CpG at any time points and concentrations examined.

To examine whether the maleimide-activation of CTB or the thiolation of CpG ODN has any effect on the observed chemokine responses by CTB:CpG conjugates, PBMCs were incubated with CTB, CTB-maleimide, CpG ODN or thiol CpG ODN and then the CC chemokine response was examined. Similar levels of MIP-1α, MIP-1β and RANTES were detected in the supernatants of PBMCs treated with either CTB or maleimide-activated CTB. Similarly, PBMCs treated with CpG ODN or thiolated CpG ODN gave rise to production of the same levels of these chemokines at any time point tested (figure 13). Thus, chemical modification of CTB (maleimide activation) and CpG ODN (thiolation) had no additional effects on CTB- or CpG ODN-induced chemokine responses.

### References

Baek K. H., Ha S. J. and Sung Y. C. (2001) A novel function of phosphorothioate oligodeoxynucleotides as chemoattractants for primary macrophages. J. Immunol. 167, 2847-2854.
Ban E., Dupre L., Hermann E., Rohn W., Vendeville C., Quatannens B., Ricciardi-Castagnoli P., Capron A. and Riveau G. (2000) CpG motifs induce Langerhans cell migration in vivo. Intl. Immunol. 12, 737-745.
Elkins K., Rhinehart-Jones T., Stibitz S., Conover J. and Klinman D. (1999) Bacterial DNA containing CpG motifs stimulates lymphocyte-dependent protection of mice against lethal infection with intracellular bacteria. J. Immunol. 162, 2291-2298.
Gramzinski R., Doolan D., Sedegah M., Davis H., Krieg A. and Hoffman S. (2001) Interleukin-12- and gamma interferon-dependent protection against malaria conferred by CpG oligodeoxynucleotide in mice. Infect Immun. 69, 1643-1649.
Harandi A. M., Eriksson K. and Holmgren J. (2003) A protective role of locally administered immunostimulatory CpG oligodeoxynucleotide in a mouse model of genital herpes infection. J. Viral. 77, 953-962.
Holmgren J., Harandi A. M. and Czerkinsky C. (2003) Mucosal adjuvants and anti-infection and anti-immunopathology vaccines based on cholera toxin, cholera toxin B subunit and CpG DNA. Expert Rev. Vaccines 2, 205-217.
Kinghom G. R. (1994) Epidemiology of genital herpes. J. Int. Med. Res. 22, 14A-23A.
Krieg A. (2001) Immune effects and mechanisms of action of CpG motifs. Vaccine 19, 618-622.
Krieg A. and Davis H. (2001) Enhancing vaccine with immune stimulatory CpG DNA. Curr. Opin. Mol. Ther. 3, 15,24.
Krieg A., Love-Homan L., Yi A. and Harty J. (1998) CpG DNA induces sustained IL-12 expression in vivo and resistance to Listeria monocytogenes challenge. J. Immunol. 161, 2428-2434.
Krieg A., Yi A., Matson S., Waldschmidt T., Bishop G., Teasdale R., Koretzky G. and Klinman D. (1995) CpG motifs in bacterial DNA trigger direct B-cell activation. Nature 374, 546-549.
Krieg A. M. (2002) CpG motifs in bacterial DNA and their immune effects. Annu. Rev. Immunol. 20, 709-760.
Lipford G., Sparwasser T., Bauer M., Zimmermann S., Koch E., Heeg K. and Wagner H. (1997) Immunostimulatory DNA: sequence-dependent production of potentially harmful or useful cytokines. Eur. J. Immunol. 27, 3420-3426.
Nahmias A. J., Lee F. K. and Beckman-Nahmias S. (1990) Sero-epidemiological and - sociological patterns of herpes simplex virus infection in the world. Scand. J. Infect. Dis. Suppl. 69, 19-36.
Roman M., Martin-Orozco E., Goodman J., Nguyen M., Sato Y., Ronaghy A., Kornbluth R., Richman D., Carson D. and Raz E. (1997) Immunostimulatory DNA sequences function as T helper-1-promoting adjuvants. Nat. Med. 3, 849-854.
Sano K., Shirota H., Terui T., Hattori T. and Tamura G. (2003) Oligodeoxynucleotides without CpG motifs work as adjuvant for the induction of Th2 differentiation in a sequence-independent manner. J Immunol, 2367-2373.
Seth P., Rawls W. E., Duff R., Rap F., Adam E. and Melnick J. L. (1974) Antigenic differences between isolates of herpesvirus type 2. Intervirology 3, 1-14.
Shirota H., Sano K., Hirasawa N., Terui T., Ohuchi K., Hattori T., Shirato K. and Tamura G. (2001) Novel roles of CpG oligodeoxynucleotides as a leader for the sampling and presentation of CpG-tagged antigen by dendritic cells. J. Immunol. 167, 66-74.
Shirota H., Sano K., Kikuchi T., Tamura G. and Shirato K. (2000) Regulation of murine airway eosinophilia and Th2 cells by antigen-conjugated CpG oligodeoxynucleotides as a novel antigen-specific immunomodulator. J. Immunol. 164, 5575-5582.
Sparwasser T., Koch E. S., Vabulas R. M., Heeg K., Lipford G. B., Ellwart G. B. and Wagner H. (1998) Bacterial DNA and immunostimulatory CpG oligonucleotides trigger maturation and activation of murine dendritic cells. Eur. J. Immunol. 28, 2045-2054.
Sparwasser T., Miethke T., Lipford G., Borschert K., Hacker H., Heeg K. and Wagner H. (1997a) Bacterial DNA causes septic shock. Nature 386, 336-337.
Sparwasser T., Miethke T., Lipford G., Erdmann A., Hacker H., Heeg K. and Wagner H. (1997b) Macrophages sense pathogens via DNA motifs: induction of tumor necrosis factor-alpha-mediated shock. Eur. J. Immunol. 27, 1671-1679.
Stunz L. L., Lenert P., Peckham D., Yi A. K., Haxhinasto S., Chang M., Krieg A. M. and F A. R. (2002) Inhibitory oligonucleotides specifically block effects of stimulatory CpG oligonucleotides in B cells. Eur. J. Immunol. 32, 1212-1222.
Whitley R. J. (1991) Perinatal herpes simplex virus infections. Rev. Med. Virol. 1, 101-110.
Yamada H., Gursel I., Takeshita F., Conover J., Ishii K. J., Gursel M., Takeshita S. and Klinman D. M. (2002) Effect of suppressive DNA on CpG-induced immune activation. J. Immunol. 169, 5590-5594.
Yamamoto S., Yamamoto T., Kataoka T., Kuramoto E., Yano O. and Tokunaga T. (1992a) Unique palindromic sequences in synthetic oligonucleotides are required to induce IFN -gamma and augment IFN-gamma-mediated natural killer activity. J. Immunol. 148, 4072-4076.
Yamamoto S., Yamamoto T., Shimada S., Kuramoto E., Yano O., Kataoka T. and Tokunaga T. (1992b) DNA from bacteria, but not from vertebrates, induces interferons, activates natural killer cells and inhibits tumor growth. Microbiol. Immunol. 36, 983-997.
Yi A., Klinman D., Martin T., Matson S. and Krieg A. (1996) Rapid immune activation by CpG motifs in bacterial DNA. Systemic induction of IL-6 transcription through an antioxidant-sensitive pathway. J. Immunol. 157, 5394-5402.
Zhu F.-G., Reich C. F. and Pisetsky D. S. (2002) Inhibition of murine dendritic cell activation by synthetic phosphorothioate oligodeoxynucleotides. J. Leuk. Biol. 72, 1154-1163.
Zimmermann S., Egeter O., Hausmann S., Lipford G., Röcken M., Wagner H. and Heeg K. (1998) CpG oligodeoxynucleotides trigger protective and curative Th1 responses in lethal murine leishmaniasis. J. Immunol. 160, 3627-3630.

## Claims

1. A bifunctional composition, optionally in combination with a specific antigen, comprising a protein binding to specific receptors on mammalian cell surfaces selected from the group consisting of cholera toxin B subunit (CTB), *Escherichia coli* heat labile enterotoxin B subunit (LTB), and proteins or protein derivatives that react with antiserum to CTB or LTB or bind to GM1 ganglioside,
conjugated with
an intracellularly effective immunomodulating nucleic acid component containing at least one unmethylated 5'-cytosine, guanine-3' dinucleotide (CpG) selected from an oligonucleotide or a polynucleotide with at least 6 bases or base pairs and with either a natural phosphodiester backbone, a phosphothioate backbone or a chimera of phosphodiester and phosphothioate backbone.

2. The composition according to claim 1, wherein the immunomodulating nucleic acid component is selected from the group consisting of an CpG-containing immunostimulatory oligo- or polynucleotide (ISS) selected from single stranded and double stranded DNA, single and double stranded RNA and modified polynucleotides.

3. The composition according to claim 2, wherein the ISS is derived from a microbial genome.

4. The composition according to claim 3, wherein the microbial genome is *Herpes simplex* virus genome.

5. The composition according to any one of claims 2-4, wherein the ISS is 5'-purine purine(pyrimidine) CG pyrimidine pyrimidine-3' (5'-Pu Pu(Py)CGPy Py-3').

6. The composition according to any one of claims 2- 5, wherein the ISS is selected from 5'-GACGTT-3' and 5'-GTCGTT-3'.

7. The composition according to claim 1, wherein the specific antigen is selected from the group consisting of synthetic or natural pathogen-derived antigens, mammalian tissue-derived antigens, allergens and host-derived antigens.

8. A bifunctional composition according to any one of claims 1 - 7 for use as a medicament.

9. Use of a bifunctional composition according to any one of the claims 1 - 6 for the manufacture of a pharmaceutical preparation for the prophylactic or therapeutic treatment of tumors, infections, graft rejections, immunosuppressive states, autoimmune diseases and allergies.

10. Use of a composition according to claim 7, wherein the pharmaceutical preparation is antigen-free and is for local treatment of epithelial tumors or non-respiratory epithelial infections in a mammal.

11. Use of a composition according to claim 7 for the manufacture of a pharmaceutical preparation for immunoprophylaxis, immunotherapy or induction of tolerance.

12. Use of composition according to claims 7 for treatment *ex vivo* of an antigen-presenting cell for subsequent manufacture of a pharmaceutical preparation for infusion into a mammal for vaccination or immunotherapy purposes.

## Patentansprüche

1. Bifunktionale Zusammensetzung, wahlweise in Kombination mit einem spezifischen Antigen, ein Protein enthaltend, das an spezifische Rezeptoren auf Zelloberflächen von Säugetieren bindet und ausgewählt ist aus der Gruppe bestehend aus der B-Untereinheit des Choleratoxins (CTB), der B-Untereinheit des hitzelabilen Enterotoxins von *Escherichia coli* (LTB) und aus Proteinen oder Proteinderivaten, die mit Antiserum gegen CTB oder LTB reagieren oder an GM1-Gangliosid binden,
konjugiert mit
einer intrazellulär wirkenden immunmodulierenden Nukleinsäurekomponente, die mindestens ein unmethyliertes 5'-Cytosin-Guanin-3'-Dinukleotid (CpG) enthält, ausgewählt aus einem Oligonukleotid oder einem Polynukleotid mit mindestens 6 Basen oder Basenpaaren und entweder einem natürlichen Phosphodiesterrückgrat, einem Phosphothioatrückgrat oder einem chimären Phosphodiester- und Phosphothioatrückgrat.

2. Zusammensetzung nach Anspruch 1, wobei die immunmodulierende Nukleinsäurekomponente ausgewählt ist aus der Gruppe bestehend aus einem CpG enthaltenden immunstimulierenden Oligo- oder Polynukleotid (ISS), das ausgewählt ist aus einsträngiger und doppelsträngiger DNA, einsträngiger und doppelsträngiger RNA und modifizierten Polynukleotiden.

3. Zusammensetzung nach Anspruch 2, wobei die ISS von einem mikrobiellen Genom abgeleitet ist.

4. Zusammensetzung nach Anspruch 3, wobei das mikrobielle Genom das Genom des *Herpes-simplex*-Virus ist.

5. Zusammensetzung nach einem der Ansprüche 2 - 4, wobei die ISS 5'-Purin-Purin(Pyrimidin) CG Pyrimidin Pyrimidin-3' (5'-Pu Pu (Py) CGPy Py-3') ist.

6. Zusammensetzung nach einem der Ansprüche 2 - 5, wobei die ISS aus 5'-GACGTT-3' und 5'-GTCGTT-3' ausgewählt ist.

7. Zusammensetzung nach Anspruch 1, wobei das spezifische Antigen ausgewählt ist aus der Gruppe bestehend aus synthetischen oder natürlichen von Erregern abgeleiteten Antigenen, von Gewebe von Säugetieren abgeleiteten Antigenen, Allergenen und von Wirtsorganismen abgeleiteten Antigenen.

8. Bifunktionale Zusammensetzung nach einem der Ansprüche 1 - 7 zur Verwendung als Medikament.

9. Verwendung einer bifunktionalen Zusammensetzung nach einem der Ansprüche 1 - 6 für die Herstellung eines pharmazeutischen Präparats für die prophylaktische oder therapeutische Behandlung von Tumoren, Infektionen, Abstossungen, immunsuppressiven Zuständen, Autoimmunerkrankungen und Allergien.

10. Verwendung einer Zusammensetzung nach Anspruch 7, wobei das pharmazeutische Präparat antigenfrei ist und zur lokalen Behandlung von Epitheltumoren oder nichtrespiratorischen Epithelinfektionen bei einem Säugetier dient.

11. Verwendung einer Zusammensetzung nach Anspruch 7 zur Herstellung eines pharmazeutischen Präparats für die Immunprophylaxe, Immuntherapie oder Toleranzinduktion.

12. Verwendung der Zusammensetzung nach Anspruch 7 für die Ex-vivo-Behandlung einer antigenpräsentierenden Zelle zur nachfolgenden Herstellung eines pharmazeutischen Präparats zur Infusion in ein Säugetier zwecks Impfung oder Immuntherapie.

## Revendications

1. Composition bifonctionnelle, optionnellement en combinaison avec un antigène spécifique, comprenant une protéine se liant à des récepteurs spécifiques sur des surfaces de cellules de mammifères choisie parmi le groupe comprenant la sous-unité B de la toxine du choléra (CTB), la sous-unité B de l'entérotoxine thermolabile d'*Escherichia coli* (LTB) et de protéines ou dérivés de protéines réagissant avec un antisérum contre CTB ou LTB ou se liant au ganglioside GM1,
conjuguée avec
un composant d'acide nucléique immunomodulateur intracellulairement efficace comprenant au moins un dinucléotide 5'-cytosine, guanine-3' (CpG) non méthylé choisi parmi un oligonucléotide ou un polynucléotide ayant au moins 6 bases ou paires de bases et ayant soit un squelette phosphodiester naturel, un squelette phosphothioate ou une chimère d'un squelette phosphodiester et phosphothioate.

2. Composition selon la revendication 1, où le composant d'acide nucléique immunomodulateur est choisi parmi le groupe comprenant un oligo- ou polynucléotide immunostimulateur (ISS) contenant CpG, choisi parmi l'ADN monocaténaire et bicaténaire, l'ARN monocaténaire et bicaténaire et les polynucléotides modifiés.

3. Composition selon la revendication 2, où l'ISS est dérivée d'un génome microbien.

4. Composition selon la revendication 3, où le génome microbien est le génome du virus *Herpes simplex*.

5. Composition selon l'une quelconque des revendications 2 à 4, où l'ISS est la séquence 5'-purine purine(pyrimidine) CG pyrimidine pyrimidine-3' (5'-Pu Pu(Py)CGPy Py-3').

6. Composition selon l'une quelconque des revendications 2 à 5, où l'ISS est choisie parmi 5'-GACGTT-3' et 5'-GTCGTT-3'.

7. Composition selon la revendication 1, où l'antigène spécifique est choisi parmi le groupe comprenant des antigènes dérivés d'agents pathogènes, synthétiques ou naturels, des antigènes dérivés de tissu de mammifères, des allergènes et des antigènes dérivés d'organismes hôtes.

8. Composition bifonctionnelle selon l'une quelconque des revendications 1 à 7 pour utilisation en tant que médicament.

9. Utilisation d'une composition bifonctionnelle selon l'une quelconque des revendications 1 à 6 pour la fabrication d'une préparation pharmaceutique pour le traitement prophylactique ou thérapeutique de tumeurs, d'infections, de rejets de greffes, d'états immunosuppressifs, de maladies auto-immunes et d'allergies.

10. Utilisation d'une composition selon la revendication 7, où la préparation pharmaceutique est exempte d'antigènes et est destinée au traitement local de tumeurs épithéliales ou d'infections épithéliales non respiratoires d'un mammifère.

11. Utilisation d'une composition selon la revendication 7 pour la fabrication d'une préparation pharmaceutique pour l'immunoprophylaxie, l'immunothérapie ou l'induction de tolérance.

12. Utilisation de la composition selon la revendication 7 pour le traitement *ex vivo* d'une cellule présentant l'antigène pour la fabrication subséquente d'une préparation pharmaceutique pour infusion dans un mammifère à des fins de vaccination ou d'immunothérapie.
